(19) Europäisches Patentamt
European Patent Office
Office européen des brevets

(11) Veröffentlichungsnummer: **0 000 534**
**B1**

(12) **EUROPÄISCHE PATENTSCHRIFT**

(45) Veröffentlichungstag der Patentschrift: **03.02.82**

(51) Int. Cl.³: **C 07 D 303/04,**
C 07 D 301/14

(21) Anmeldenummer: **78100420.5**

(22) Anmeldetag: **18.07.78**

# (54) Verfahren zur Herstellung von Vinyloxiranen.

(30) Priorität: **29.07.77 DE 2734242**

(43) Veröffentlichungstag der Anmeldung:
**07.02.79 Patentblatt 79/3**

(45) Bekanntmachung des Hinweises auf die Patenterteilung:
**03.02.82 Patentblatt 82/5**

(84) Benannte Vertragsstaaten:
**BE CH DE FR GB NL**

(56) Entgegenhaltungen:
**FR - A - 2 300 085**
**FR - A - 2 309 551**
**FR - A - 2 309 552**
**FR - A - 2 369 273**
**FR - A - 2 369 274**
**GB - A - 846 534**

(73) Patentinhaber: **BAYER AG**
**Zentralbereich Patente, Marken und Lizenzen**
**D-5090 Leverkusen 1, Bayerwerk (DE)**

(72) Erfinder: **Rauleder, Gebhard, Dr.**
**Cheruskerstrasse 31**
**D-4000 Düsseldorf (DE)**
Erfinder: **Seifert, Hermann, Dr.**
**Ruwergasse 4**
**D-5000 Köln (DE)**
Erfinder: **Waldmann, Helmut, Dr.**
**Carl-Rumpff-Strasse 59**
**D-5090 Leverkusen 1 (DE)**
Erfinder: **Schwerdtel, Wulf, Dr.**
**Hegelstrasse 9**
**D-5090 Leverkusen 1 (DE)**
Erfinder: **Swodenk, Wolfgang, Dr.**
**Auf dem Broich 5**
**D-5068 Odenthal 1 (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 000 534 B1

Courier Press, Leamington Spa, England.

## Verfahren zur Herstellung von Vinyloxiranen

Die vorliegende Erfindung betrifft ein Verfahren zur Herstellung von Vinyloxiranen aus Polyenen und Percarbonsäuren.

Vinyloxirane haben als Monomere aufgrund ihrer bifunktionellen Struktur eine besondere Bedeutung. Sie finden deshalb sowohl auf dem Gebiet der Lacke und Kunststoffe als auch als organische Zwischenprodukte Verwendung. Ein technischer Einsatz der Vinyloxirane ist in größerem Maße bisher nicht erfolgt, da noch kein befriedigendes technisches Verfahren zu ihrer Herstellung zur Verfügung gestanden hat.

Es sind bereits viele Methoden zur Umwandlung von Olefinen in Oxirane bekannt, und es ist auch versucht worden, diese Methoden zur Herstellung von Vinyloxiranen anzuwenden. So wird in der USA—PS 3 232 957 vorgeschlagen, Olefine mit molekularem Sauerstoff in flüssiger Phase in die Epoxide umzuwandeln. In Spalte 10, Zeile 15, Beispiel 8 der genannten Patentschrift wird die Umsetzung von Butadien mit Sauerstoff bei 150°C in Dicyclopropylketon als Lösungsmittel beschrieben. Die erreichte Ausbeute an Vinyloxiran war jedoch niedrig; sie betrug nur 25%. Auch die Verwendung von Acetophenon anstelle von Dicyclopropylketon als Lösungsmittel erbrachte nur eine geringfügig höhere Ausbeute an Vinyloxiran; sie betrug nur 28% (USA—PS 3 232 957, Spalte 12, Zeile 15).

Die seit langem zur Herstellung von Oxiranen aus Olefinen bekannte Halogenhydrinmethode wurde auch zur Herstellung von Vinyloxiranen angewandt. Beispielsweise wird in der bekanntgemachten Japanischen Patentanmeldung 23 545/64 die Herstellung von Isoprenoxid aus Isoprenmonochlorhydrin und Alkalihydroxid beschrieben. Diese Methode besitzt jedoch den Nachteil der Bildung unerwünschter chlorierter Nebenprodukte und umweltbelastender Salzabfälle (Ullmanns Encyklopädie d. techn. Chemie, Bd. 10, S. 565, linke Spalte, Zeile 1 ff.; DAS 1 543 174, Spalte 2, Zeile 15 ff.).

Eine andere Methode zur Herstellung von Epoxyverbindungen beruht auf der Umsetzung von Olefinen mit Wasserstoffperoxid in Gegenwart von Katalysatoren. Die Anwendung dieser Methode zur Erzeugung von Vinyloxiranen aus Polyenen und Wasserstoffperoxid wird in der DAS 2 012 049, S. 15, Zeile 7, Beispiel 18, insbesondere Tabelle I (Fortsetzung) beschrieben. Darin wird über die Umsetzung von Butadien mit 70%-igem Wasserstoffperoxid in Gegenwart von Trimethylzinnhydroxid und dem Molybdänsalz des Acetylacetons als Katalysator berichtet. Als Lösungsmittel wurde Äthanol verwendet. Aber selbst nach 20-stündigem Rühren bei 40°C wurde Vinyloxiran nur in einer Ausbeute von 21,7%, bezogen auf eingesetztes Wasserstoffperoxid, erhalten.

Ein weiteres Syntheseprinzip beruht auf der Umsetzung von Olefinen mit organischen Hydroperoxiden mit Hilfe von Katalysatoren. Die Anwendung dieses Syntheseprinzips zur Herstellung von Vinyloxiranen aus Polyenen und tert.-Butylhydroperoxid oder Cumolhydroperoxid in Gegenwart von Molybdänhexacarbonyl als Katalysator wird von M. N. Sheng und J. G. Zajacek beschrieben (J. Org. Chem. *35*, No. 6, (1970) S. 1840, Tabelle I; USA—PS 3 538 124). Obwohl für die Umwandlung von Butadien, Isopren und Piperylen in die Monoepoxide Ausbeuten von 84—90% erhalten werden, hat dieses Syntheseprinzip doch den entscheidenden Nachteil, daß das organische Hydroperoxid ROOH, wobei R einen niedermolekularen organischen Rest wie tert.-Butyl oder Cumyl bedeutet, gemäß nachstehender Gleichung während der Reaktion in den entsprechenden Alkohol ROH umgewandelt wird:

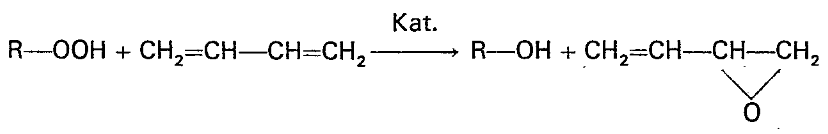


Es fällt somit der dem eingesetzten Hydroperoxid (ROOH) entsprechende Alkohol als Koppelprodukt an, was eine Abhängigkeit der Wirtschaftlichkeit dieses Verfahrens von der Verwertbarkeit dieses Alkohols (ROH) mit sich bringt. Falls dieser Alkohol nicht verwendet werden kann, muß er über mehrere Verfahrensstufen in das entsprechende Hydroperoxid (ROOH) zurückverwandelt werden.

Eine weitere Methode zur Herstellung von Vinyloxiran aus Butadien wird in der DAS 1 593 306 auf Seite 8 im Beispiel 3 beschrieben. Hierin wird Butadien mit Tri-(cyclohexylperoxy-) boran bei einem Druck von 20 bar und einer Temperatur von 100°C umgesetzt. Die destillative Aufarbeitung des Reaktionsgemisches ergab eine zwischen 70°C und 126°C siedende Fraktion, die Vinyloxiran in einer Menge enthielt, die einer Ausbeute von 81,7% entsprach. Offensichtlich treten bei dieser Methode Probleme bei der Abtrennung des Vinyloxirans aus dem Reaktionsgemisch auf. Auch die angegebene Ausbeute an Vinyloxiran ist unbefriedigend.

Die Umsetzung von Polyenen mit Percarbonsäuren beschreiben erstmals Pummerer und Reindel 1933 (R. Pummerer und W. Reindel, Ber. dtsch. chem. Ges. *66*, S. 334—339 (1933)). Auf Seite 335, Zeile 16 ff. berichten sie über die Epoxidation von Isopren mit Perbenzoesäure in Äthylchlorid als Lösungsmittel. Sie erhalten als einziges Produkt 2-Methyl-2-vinyloxiran in einer Ausbeute von 30 bis 40%. Bei der Umsetzung von Butadien mit Perbenzoesäure erreichen sie für Vinyloxiran eine Ausbeute von etwa 40%.

Eine wesentliche Verbesserung konnte auch von F. C. Frostick et. al. (J. Am. Chem. Soc., *81*, 3354, Tabelle IV) nicht erreicht werden. Bei der Umsetzung von Butadien mit Peressigsäure, die durch Oxidation von Acetaldehyd hergestellt wurde, in Äthylacetat oder Aceton als Lösungsmittel erzielte er eine Ausbeute an Vinyloxiran von 56%.

Auch die Anwendung spezieller verfahrenstechnischer Maßnahmen wie sie in der Deutschen PS 1 216 506 zur Durchführung von Epoxidationen vorgeschlagen werden, bringen keine befriedigenden Ausbeuten. Im Beispiel 3, Spalte 8, Zeile 60 ff. dieser Patentschrift wird die Umsetzung von Butadien mit Peressigsäure in Äthylacetat beschrieben. Vinyloxiran wird dabei in 68%iger Ausbeute erhalten.

Die Verwendung von Peressigsäure, die ebenfalls durch Oxidation von Acetaldehyd hergestellt wurde, in Essigsaüre als Lösungsmittel wird in der Japanischen PS I 74 046—284, und die Verwendung von Perisobuttersäure, die durch Oxidation von Isobutyraldehyd erhalten wurde, in Isobuttersäure als Lösungsmittel wird in der Japanischen PS I 74 040—202 zur Epoxidation von Butadien vorgeschlagen. Die Verwendung von Carbonsäuren als als Lösungsmittel bei der Herstellung von Oxiranen besitzt aber den Nachteil, daß viele Oxirane mit Carbonsäuren unter Ringöffnung zu den Acylaten reagieren, (D. Swern, "Organic Peroxides", Wiley Interscience, 1971, Vol. 2, S. 376, Zeile 10 ff.), was zu erheblichen Ausbeuteverlusten führen kann.

In der DAS 1 190 926 wird die Percarbonsäure bei der Umsetzung mit Butadien "in situ" erzeugt. Die erreichte Ausbeute ist jedoch auch hierbei nicht befriedigend. In Spalte 15, Zeile 5 ff., wird die Umsetzung von Butadien bei 73—76°C in Äthylacetat als Lösungsmittel mit Sauerstoff in Gegenwart von n-Butyraldehyd und Kobaltnaphthenat als Katalysator beschrieben. Die Ausbeute an Epoxid wird mit 55% angegeben. Davon entfallen 45% auf Butadienmonoxid und 10% auf Butadiendioxid.

Eine weitere Möglichkeit zur Herstellung von Vinyloxiranen wird in der Britischen PS 735 974 beschrieben. Darin wird im Beispiel 13, Spalte 8, Zeile 31 ff., die Umsetzung von Butadien mit durch Oxidation von Acetaldehyd hergestelltem Acetaldehydmonoperacetat in Aceton als Lösungsmittel bei 70°C beschrieben. Vinyloxiran wird jedoch nur in einer Ausbeute von 33% erhalten.

Ein anderes Syntheseprinzip zur Epoxidation von Polyenen wird in der USA—PS 3 053 856 beschrieben. Hierbei wird die Umsetzung von Butadien mit Wasserstoffperoxid in Gegenwart von Acetonitril durchgeführt, wobei Vinyloxiran als Hauptprodukt erhalten wird (Beispiel XII). Allerdings muß bei diesem Verfahren in Kauf genommen werden, daß man das aus dem Nitril während der Reaktion gebildete Carbonsäureamid als Koppelprodukt erhält, das entweder einer geeigneten Verwendung zugeführt oder aber einer Dehydratisierung unterworfen werden muß, um in den Prozeß zurückgeführt werden zu können.

Schließlich ist aus der GB—PS 846 534 eine Umsetzung von Butadien mit Peressigsäure im Molverhältnis 1,23:1 bekannt. Die sonstigen Angaben zu dieser Umsetzung reichen nicht aus, um dem Fachmann eine Lehre zum Handeln zu geben, wie er in hohen Ausbeuten Butadienmonoxid erhalten kann. Auch ist aus dieser Schrift nicht bekannt, daß im Überschuß eingesetztes Butadien wiedergewonnen werden kann. Wegen der hohen Raektions- und Polymerisationsfähigkeit des Butadiens und mangels entsprechender Angaben über den Verbleib des im Überschuß eingesetzten Butadiens, vermutet der Fachmann, daß dieses nicht wiedergewonnen werden kann und der Einsatz von Butadien in größeren Überschüssen keine Vorteile bietet. Außerdem fallen gemäß der in dieser Schrift beschriebenen Arbeitsweise große Mengen umweltbelastender Abwässer an, weshalb es für eine technische Anwendung unvorteilhaft ist. Diese Schrift enthält auch keine Angaben über die Qualität der eingesetzten Persäure.

Es wurde nun ein Verfharen zur Herstellung von Vinyloxiran und substituierten Vinyloxiran aus Polyenen und Percarbonsäuren gefunden, das dadurch gekennzeichnet ist, daß man ein Polyen der Formel

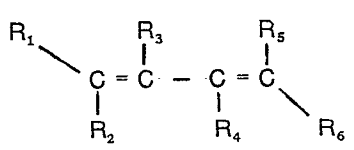

worin $R_1$, $R_2$, $R_4$, $R_5$ und $R_6$ unabhängig voneinander Wasserstoff, $C_1$- bis $C_4$-Alkyl, Vinyl, $C_3$- bis $C_7$-Cycloalkyl oder Phenyl bedeuten und worin $R_1$ mit $R_3$, $R_1$ mit $R_4$ oder $R_1$ mit $R_5$ einen carbocyclischen Ring mit bis zu 12 Kohlenstoffatomen bilden können,

mit einer Lösung einer 3 bis 4 Kohlenstoffatome enthaltenden Percarbonsäure in einem aromatischen Kohlenwasserstoff mit 6 bis 12 Kohlenstoffatomen oder in einem chlorierten, 1 bis 18 Kohlenstoffatome enthaltenden Kohlenwasserstoff, die bis zu 5 Gew.-% Wasser, bis zu 2 Gew.-% Wasserstoffperoxid und weniger als 50 ppm Mineralsäure enthält, bei einem Molverhältnis von Polyen zu Percarbonsäure wie 1,5:1 bis 5:1 und bei einer Temperatur von —20°C bis +100°C umsetzt.

Im Rahmen der Verbindungen der Formel (I) kommen beispielsweise Verbindungen der folgenden Formel (II—V) besonders in Betracht:

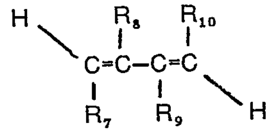

(II)

worin $R_7$, $R_8$, $R_9$ und $R_{10}$ unabhängig voneinander Wasserstoff, $C_1$- bis $C_4$-Alkyl, Vinyl, $C_5$- bis $C_7$-Cycloalkyl oder Phenyl bedeuten und worin $R_7$ mit $R_8$, $R_7$ mit $R_9$ oder $R_7$ mit $R_{10}$ einen carbocyclischen Ring mit bis zu 12 Kohlenstoffatomen bilden können.

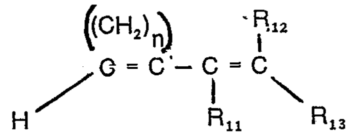

(III)

worin $R_{11}$, $R_{12}$ und $R_{13}$ unabhängig voneinander Wasserstoff, $C_1$- bis $C_4$-Alkyl, Vinyl, $C_1$- bis $C_5$-Cycloalkyl oder Phenyl bedeuten und n für eine ganze Zahl von 3 bis 6 steht;

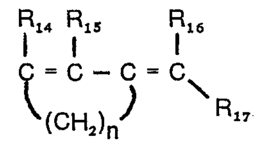

(IV)

worin $R_{14}$, $R_{15}$, $R_{16}$ und $R_{17}$ unabhängig voneinander Wasserstoff, $C_1$- bis $C_4$-Alkyl, Vinyl, $C_5$- bis $C_7$-Cycloalkyl oder Phenyl bedeuten und n für eine ganze Zahl von 2 bis 5 steht;

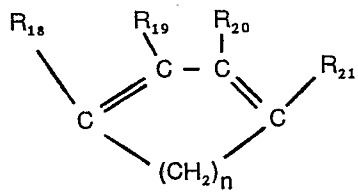

(V)

wobei $R_{18}$, $R_{19}$, $R_{20}$ und $R_{21}$ unabhängig voneinander Wasserstoff, $C_1$- bis $C_4$-Alkyl, Vinyl, $C_5$- bis $C_7$-Cycloalkyl oder Phenyl bedeuten und n für eine ganze Zahl von 1 bis 4 steht.

Im einzelnen seien als Polyene genannt:

Butadien, Isopren, Piperylen, Cyclopentadien, Methyl-cyclopentadien, 2,4-Dimethyl-1,3-butadien, 1,3-Dimethyl-1,3-butadien, 2,4-Hexadien, 1,3-Hexadien, 1,3,5-Hexatrien, Dimethyl-cyclopentadien, 2-Methyl-1,3-pentadien, 3-Methyl-1,3-pentadien, 4-Methyl-1,3-pentadien, 2-Äthyl-1,3-butadien, 1,3-Cyclohexadien, Methylen-2-cyclopenten, Methylen-2-cyclohexen, 3,4-Dimethyl-1,3-pentadien, 2,4-Dimethyl-1,3-pentadien, 2-Methyl-2,4-hexadien, 2-Methyl-1,3-hexadien, 2-Methyl-1,3,5-hexatrien, 3-Methyl-1,3-hexadien, 4-Methyl-1,3-hexadien, 3-Methyl-1,3,5-hexatrien, Methyl-1,3-cyclohexadien, 1,3-Cycloheptadien, 1,3,5-Cycloheptatrien, Dimethyl-1,3-cyclohexadien, 1-Vinyl-1-cyclohexen, Methyl-1,3-cycloheptadien, 2,5-Dimethyl-1,3-hexadien, 2,5-Dimethyl-2,4-hexadien, 2,5-Dimethyl-1,3,5-hexatrien, 2-Äthyl-1,3-hexadien, 2-Äthyl-1,3,5-hexatrien, 1,3-Cyclooctadien, Cyclooctatetraen, 2,4-Dimethyl-1,3,5-hexatrien, 2,4-Dimethyl-1,3-hexadien, 5,5-Dimethyl-1,3-hexadien, 5,5-Dimethyl-1,3,5-hexatrien, 1,3,7-Octatrien, 2-Methyl-1,3,7-octatrien, 2-Methyl-1,5,7-octatrien, Methyl-1,3-cyclooctadien, 1-Cyclopentyl-1,3-butadien, 2-Cyclopentyl-1,3-butadien, 1,2,3,4-Tetramethyl-cyclopentadien, 2,6-Dimethyl-1,3,7-octatrien, 3,7-Dimethyl-1,3,7-octatrien, 1-Cyclohexyl-1,3-butadien, 2-Cyclohexyl-1,3-butadien, 1-Phenyl-1,3-butadien, 2-Phenyl-1,3-butadien, 1,3,9,11-Dodecatetraen, 1,2,3,4-Tetramethyl-cyclooctatetraen.

Besonders geeignet zur Umsetzung mit Percarbonsäuren gemäß dem erfindungsgemäßen Verfahren sind Polyene der Formel

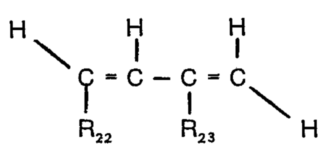

(VI)

worin $R_{22}$ und $R_{23}$ unabhängig voneinander Wasserstoff, Methyl, Äthyl, Vinyl oder Phenyl bedeuten.

Im einzelnen seien als Verbindungen der Formel VI beispielsweise genannt:

Butadien, Isopren, Piperylen, 1,3-Hexadien, 1,3,5-Hexatrien, 2-Äthyl-1,3-butadien, 1,3-Dimethyl-1,3-butadien, 2,4-Hexadien, 1-Phenyl-1,3-butadien, 2-Phenyl-1,3-butadien, 1-Methyl-1,3-hexadien, 1-Methyl-1,3,5-hexadien, 2-Methyl-1,3-pentadien, 2-Methyl-1,3-hexadien, 2-Methyl-1,3,5-hexatrien, 2-Äthyl-1,3-hexadien, 2-Äthyl-1,3,5-hexatrien, 2-Phenyl-1,3,5-hexatrien, 2-Phenyl-1,3-hexadien, 2-Phenyl-1,3-pentadien.

Ganz besonders sind zur Umsetzung mit Percarbonsäuren nach dem erfindungsgemäßen Verfahren Butadien, Isopren, Piperylen, Cyclopentadien, 1,3,5-Hexatrien und 1,3,7-Octatrien geeignet.

Als Lösungsmittel können die verschiedensten chlorierten Kohlenwasserstoff verwendet werden, wie Methylenchlorid, Chloroform, Tetrachlorkohlenstoff, 1-Chloräthan, 1,2-Dichloräthan, 1,1-Dichloräthan, 1,1,2,2-Tetrachloräthan, 1-Chlorpropan, 2-Chlorpropan, 1,2-Dichlorpropan, 1,3-Dichlorpropan, 2,3-Dichlorpropan, 1,2,3-Trichlorpropan, 1,1,2,3-Tetrachlorpropan, Butylchlorid, 1,2-Dichlorbutan, 1,4-Dichlorbutan, 2,3-Dichlorbutan, 1,3-Dichlorbutan, 1,2,3,4-Tetrachlorbutan, tert.-Butylchlorid, Amylchlorid, 1,2-Dichlorpentan, 1,5-Dichlorpentan, 1,2,3,4-Tetrachlorpentan, Cyclopentylchlorid, 1,2-Dichlorcyclopentylchlorid, Hexylchlorid, 1,2-Dichlorhexan, 1,6-Dichlorhexan, 1,2,3,4-Tetrachlorhexan, 1,2,5,6-Tetrachlorhexan, Cyclohexylchlorid, 1,2-Dichlorhexan, Chlorbenzol, Heptylchlorid, 1,2-Dichlorheptan, 1,2,1,2,3,4-Tetrachlorheptan, Cycloheptylchlorid, 1,2-Dichlorheptan, Octylchlorid, 1,2-Dichloroctan, 1,2,3,4-Tetrachloroctan, Cyclooctylchlorid oder 1,2-Dichloroctan, sowie aromatische Kohlenwasserstoffe wie Benzol, Nitrobenzol, Toluol, Äthylbenzol, Cumol, Diisopropylbenzol, Xylol oder Chlorbenzol.

Bevorzugte Lösungsmittel sind von den chlorierten Kohlenwasserstoffen Methylenchlorid, Chloroform, Tetrachlorkohlenstoff und 1,2-Dichlorpropan, von den aromatischen Kohlenwasserstoffen Benzol, Nitrobenzol, Toluol und Chlorbenzol.

Besonders bevorzugtes Lösungsmittel ist von den chlorierten Kohlenwasserstoffen, 1,2-Dichlorpropan und von den aromatischen Kohlenwasserstoffen Benzol und Chlorbenzol.

Verwendet werden können auch Lösungsmittelgemische chlorierter Kohlenwasserstoffe und Lösungsmittelgemische aromatischer Kohlenwasserstoffe. Man kann auch Gemische chlorierter und aromatischer Kohlenwasserstoffe als Lösungsmittel verwenden.

Erfindungsgemäß verwendbare Persäuren sind Perpropionsäure, Per-n-buttersäure und Perisobuttersäure. Bevorzugt verwendet werden Perpropionsäure und Perisobuttersäure. Besonders bevorzugt ist Perpropionsäure. Die Herstellung der mineralsäurefreien Persäuren in einem der genannten organischen Lösungsmittel kann z.B. nach dem in der DOS 2 262 970 beschriebenen verfahren erfolgen.

Das erfindungsgemäße Verfahren wird in einem Temperaturbereich von —20 bis 100°C durchgeführt. Bevorzugt arbeitet man bei 0—60°C, besonders bevorzugt bei 10—40°C. In Sonderfällen können auch die angegebenen Temperaturen unter- oder überschritten werden.

Neben der Arbeitsweise unter isothermen Bedingungen, d.h. Einhaltung einer einheitlichen Temperatur im gesamten Reaktionsgemisch, kann man die Umsetzung auch unter Ausbildung eines sogenannten Temperaturgradienten durchführen, der im allgemeinen mit fortschreitender Reaktion zunimmt. Man kann aber auch die Reaktion so führen, daß sich mit dem Fortschreiten der Reaktion ein Gradient fallender Temperatur ausbildet.

Das Molverhältnis von Olefin zu Persäure beträgt erfindungsgemäß 1,5:1 bis 5:1. Ganz besonders vorteilhaft ist es, ein Molverhältnis von 2,0 bis 3,0 Mol Polyen je Mol Persäure anzuwenden.

Das erfindungsgemäße Verfahren kann bei den verschiedensten Drücken durchgeführt werden. Im allgemeinen arbeitet man bei Normaldruck; das Verfahren kann jedoch auch bei Unter- oder Überdruck durchgeführt werden.

Der Wassergehalt der zur Epoxidation verwendeten Percarbonsäurelösung soll im allgemeinen möglichst niedrig sein und beträgt maximal 5 Gew.-%. Geeignet ist beispielsweise eine Percarbonsäurelösung mit einem Wassergehalt von bis zu 1 Gew.-%. Vorzugsweise verwendet man eine Percarbonsäurelösung, die weniger als 0,5 Gew.-% Wasser enthält. Besonders bevorzugt ist ein Wassergehalt von weniger als 0,1 Gew.-% z.B. ein Wassergehalt von 0,01 bis 0,1 Gew.-%.

Der Wasserstoffperoxidgehalt der angewendeten Percarbonsäurelösung soll im allgemeinen möglichst niedrig sein und beträgt maximal 2 Gew.-%. Vorteilhaft arbeitet man bei einem Gehalt von weniger als 1 Gew.-%. Besonders vorteilhaft ist es, die Umsetzung mit einer Percarbonsäurelösung durchzuführen, die einen Wasserstoffperoxidgehalt unterhalb 0,3 Gew.-% aufweist, z.B. einen Gehalt an $H_2O_2$ von 0,01 bis 0,3 Gew.-%.

Der Mineralsäuregehalt der zur Umsetzung gelangenden Percarbonsäurelösung soll möglichst niedrig sein und beträgt weniger als 50 ppm. Besonders vorteilhaft ist ein Mineralsäuregehalt von weniger als 10 ppm.

Die Durchführung der Reaktion kann diskontinuierlich oder kontinuierlich in den für Umsetzungen dieser Art üblichen Vorrichtungen wie Rührwerkskessel, Siederreaktoren, Röhrenreaktoren, Schlaufenreaktoren oder Schleifenreaktoren erfolgen.

Als Werkstoffe für die Durchführung der Verfahren können Glas, Edelstähle oder emailliertes Material verwendet werden.

Schwermetallionen im Reaktionsgemisch katalysieren die Zersetzung der Percarbonsäure. Man setzt deshalb der Percarbonsäurelösung im allgemeinen Substanzen zu, die die Schwermetallionen durch Komplexbildung inaktivieren. Bekannte Substanzen solcher Art sind Gluconsäure, Äthylendiamintetraessigsäure, Natriumsilicat, Natriumpyrophosphat, Natriumhexametaphosphat, Dinatriumdimethylpyrophosphat oder $Na_2$ (2-äthylhexyl)$_5$($P_3O_{10}$)$_2$ (DAS 1 056 596, Spalte 4, Zeile 60 ff.).

Das Polyen kann auf verschiedene Art in die für die Umsetzung verwendete Vorrichtung eingebracht werden. Man kann es gemeinsam mit der Percarbonsäurelösung in den Reaktor geben oder man führt die beiden Komponenten getrennt voneinander dem Reaktor zu. Weiterhin ist es möglich, das Olefin und die Percarbonsäurelösung an verschiedenen Stellen in die Reaktoreinheit zu leiten. Bei Verwendung mehrerer in Kaskade geschalteter Reaktoren kann es zweckmäßig sein, das gesamte Olefin in den ersten Reaktor einzubringen. Man kann aber das Olefin auch auf die verschiedenen Reaktoren aufteilen.

Die Reaktionswärme wird durch innen- oder außenliegende Kühler abgeführt. Zur Ableitung der Reaktionswärme kann die Umsetzung auch unter Rückfluß, d.h. in Siedereaktoren, durchgeführt werden.

Die Reaktion wird zweckmäßigerweise unter möglichst vollständiger Umsetzung der Percarbonsäure vorgenommen. Im allgemeinen setzt man mehr als 95 Mol.-% der Percarbonsäure um. Zweckmäßig ist es, mehr als 98 Mol.-% Persäure umzusetzen.

Bei der erfindungsgemäßen Durchführung der Reaktion zwischen dem Polyen und der Persäure gelingt es, Oxiranausbeuten von 90% der Theorie und darüber, bezogen auf eingesetzte Percarbonsäure, zu erzielen.

Die Aufarbeitung des Reaktionsgemisches erfolgt in an sich bekannter Weise, z.B. durch Destillation. Bei einer bevorzugten Durchführung des Verfahrens wird eine etwa 20 Gew.-%ige Perpropionsäurelösung in 1,2-Dichlorpropan bzw. Benzol unter Rühren zu der zweifach-molaren Menge Polyen, das auf 30°C thermostatisiert ist, gegeben. Die Perpropionsäurelösung enthält weniger als 10 ppm Mineralsäure; sie hat einen Wassergehalt, der unterhalb von 0,1% liegt und weist einen Wasserstoffperoxidgehalt von weniger als 0,3% auf. Zur Komplexierung von Schwermetallionen wurde der Perpropionsäure vor der Umsetzung etwa 0,05 Gew.-% $Na_5$($_2$-Äthylhexyl)$_5$($P_3O_{10}$)$_2$ zugesetzt. Der Fortgang und das Ende der Reaktion werden kontrolliert indem man der Reaktionslösung in zeitlichen Abständen Proben entnimmt und titrimetrisch den noch vorhandenen Gehalt an Percarbonsäure bestimmt. Nach Beendigung der Reaktion wird das Reaktionsgemisch fraktioniert.

Die folgenden Beispiele erläutern die Erfindung. Sämtliche Prozentangaben stellen, soweit nicht anderes gesagt wird, Gewichtsprozente dar.

Beispiel 1

Umsetzung von Isopren mit Perpropionsäure in 1,2-Dichlorpropan.

Zu 85,15 g (1,25 Mol) Isopren tropfte man bei 20°C unter Rühren innerhalb einer Stunde eine Lösung von 235 g (0,5 Mol) Perpropionsäure als 20%ige Lösung in 1,2-Dichlorpropan zu. Nach Zutropfende wurde noch weitere 1,5 Stunden bei dieser Temperatur gerührt; dann zeigte die titrimetrische Analyse einen Persäureumsatz von 99%. Die gaschromatographische Analyse zeigte, daß die beiden isomeren Oxirane 2-Methyl-2-vinyl-oxiran und 2-(1-Methyl äthenyl-)-oxiran im Verhältnis von 4:1 gebildet wurden. Die Gesamtselektivität der beiden Oxirane betrug 90,5%, bezogen auf eingesetzte Perpropionsäure.

Beispiel 2

Herstellung von Vinyloxiran aus Butadien und Perpropionsäure.

Zu 145,8 g (2,7 Mol) Butadien in 730 g Benzol tropfte man bei 40°C unter Rühren innerhalb einer Stunde 450 g (1 Mol) Perpropionsäure als 20%ige Lösung in Benzol zu. Nach Zutropfende wurde noch weitere 2 Stunden gerührt; dann betrug der Persäureumsatz 99%. Die Selektivität des gebildeten Vinyloxirans betrug, bezogen auf eingesetzte Persäure, 95% (GC-Analyse).

Beispiel 3

Kontinuierliche Herstellung von Vinyloxiran aus Butadien und Perpropionsäure.

20,17%ige benzolische Perpropionsäure wurde mit überschüssigen Butadien in einer vierstufigen Kesselkaskade umgesetzt. Die Reaktion wurde bei einem Druck von 2 bar durchgeführt. Butadien wurde gasförmig in den ersten Reaktor eingegeben. Der Überschuß an Butadien, bezogen auf in die Reaktion eingesetzte Perpropionsäure, betrug insgesamt 170 Mol.-%. Die in das Reaktionssystem stündlich eingegebenen Mengen betrugen 482 g Butadien und 1438 g benzolische Perpropionsäure. Der erste Reaktor dieser vierstufigen Kaskade, der ebenso wie die drei nachfolgenden Reaktionsgefäße mit einer Rührvorrichtung versehen ist und einen Inhalt von 1600 ml besitzt, wird bei einer Temperatur von 35°C, der zweite, dritte und vierte Reaktor bei einer Temperatur von jeweils 40°C betrieben. Die eingesetzte Perpropionsäure wird unter diesen Reaktionsbedingungen zu 99,8% umgesetzt. Hinter dem vierten Reaktor wurde das Reaktionsgemisch, das mit einer Menge von 1920 g pro Stunde anfiel und im Mittel die Zusammensetzung: 16,52% Butadien, 11,67% Vinyloxiran, 21,46% Propionsäure und 50,08% Benzol sowie 0,13% Wasser besaß, nach Abkühlen auf 30°C in einem Abscheider auf Normaldruck entspannt, wobei ein geringer Teil des Butadiens (37 g/h) ausgaste und wieder dem Reaktionssystem zugeführt wurde. Das nach dem Entspannen erhaltene Gemisch wurde mit 2 g/h 4-tert.-Butyl-brenzkatechin versetzt und einer ersten Destillationskolonne zugeführt. Dabei

wurde das gesamte Vinyloxiran zusammen mit Butadien und einem Teil des Benzols als Destillat erhalten. Dieses Destillat besaß im Mittel eine Zusammensetzung von 32,6% Butadien, 27,25% Vinyloxiran, 39,9% Benzol und geringen Mengen Wasser Es fiel in einer Menge von 822 g pro Stunde an und wurde anschließend in einer zweiten Destillationskolonne vom Butadien abgetrennt. Man erhielt als Destillat 268 g Butadien pro Stunde, das in das Reaktionssystem zurückgeführt wurde.

Aus dem butadienfreien Sumpfprodukt wurden in einer dritten Kolonne 224 g Vinyloxiran in einer Reinheit von 99,9% als Destillat erhalten. Das Sumpfprodukt dieser Kolonne wurde mit dem Sumpfprodukt der ersten Kolonne vereinigt und in einer vierten Kolonne aufgetrennt. Als Destillat erhielt man pro Stunde 361 g Benzol. Vom Sumpfprodukt erhielt man in einer fünften Kolonne 415 g/h Propionsäure als Destillat während am Sumpf dieser Kolonne stündlich 15 g eines Gemisches von höhersiedenden Verbindungen anfielen. Diese höhersiedenden Verbindungen bestanden im wesentlichen aus den Mono- und Dipropionaten von 3,4-Dihydroxy-1-buten. Die Ausbeute an Vinyloxiran betrug, bezogen auf die in das Reaktionsystem eingesetzte Perpropionsäure, 96,7%.

**Patentansprüche**

1. Verfahren zur Herstellung von Vinyloxiran und substituierten Vinyloxiranen aus Polyenen und Percarbonsäuren, dadurch gekennzeichnet, daß man ein Polyen der Formel :

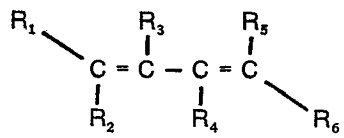

worin $R_1$, $R_2$, $R_3$, $R_4$, $R_5$ und $R_6$ unabhängig voneinander Wasserstoff, $C_1$- bis $C_4$-Alkyl, Vinyl, $C_3$- bis $C_7$-Cycloalkyl oder Phenyl bedeuten und worin $R_1$ mit $R_3$, $R_1$ mit $R_4$ oder $R_1$ mit $R_5$ einen carbocyclischen Ring mit bis zu 12 Kohlenstoffatomen bilden können,

mit einer Lösung einer 3 bis 4 Kohlenstoffatome enthaltenden Percarbonsäure in einem aromatischen Kohlenwasserstoff mit 6 bis 12 Kohlenstoffatomen oder in einem chlorierten, 1 bis 8 Kohlenstoffatome enthaltenden Kohlenwasserstoff, die bis zu 5 Gew.-% Wasser, bis zu 2 Gew.-% Wasserstoffperoxid und weniger als 50 ppm Mineralsäure enthält, bei einem Molverhältnis von Polyen zur Percarbonsäure wie 1,5:1 bis 5:1 und bei einer Temperatur von –20°C bis +100°C umsetzt.

2. Verfahren nach Anspruch 1, dadurch gekennzeichnet, daß man als Polyen ein Olefin der Formel

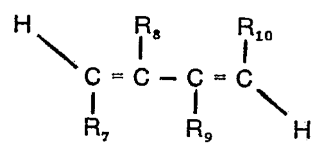

worin $R_7$, $R_8$, $R_9$ und $R_{10}$ unabhängig voneinander Wasserstoff, $C_1$- bis $C_4$-Alkyl, Vinyl, $C_5$- bis $C_7$-Cycloalkyl oder Phenyl bedeuten une worin $R_7$ mit $R_8$, $R_7$ mit $R_9$ oder $R_7$ mit $R_{10}$ einen carbocyclischen Ring mit bis zu 12 Kohlenstoffatomen bilden können, einsetzt.

3. Verfahren nach Anspruch 1 und 2, dadurch gekennzeichnet, daß man als Polyen ein Olefin der Formel

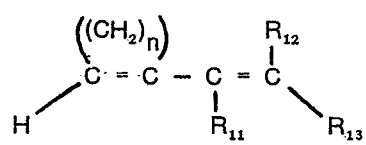

worin $R_{11}$, $R_{12}$ und $R_{13}$ unabhängig voneinander Wasserstoff, $C_1$- bis $C_4$-Alkyl, Vinyl, $C_3$- bis $C_5$-Cycloalkyl oder Phenyl bedeuten und n für eine ganze Zahl von 3 bis 6 steht, einsetzt.

4. Verfahren nach Anspruch 1 bis 3, dadurch gekennzeichnet, daß man als Polyen ein Olefin der Formel

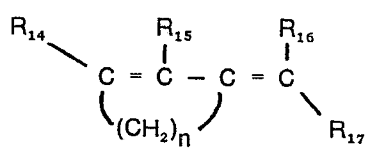

worin $R_{14}$, $R_{15}$, $R_{16}$ und $R_{17}$ unabhängig voneinander Wasserstoff, $C_1$- bis $C_4$-Alkyl, Vinyl, $C_5$- bis $C_7$-Cycloalkyl oder Phenyl bedeuten und n für eine ganze Zahl von 2 bis 5 steht, einsetzt.

5. Verfahren nach Anspruch 1 bis 4, dadurch gekennzeichnet, daß man als Polyen eine Verbindung der Formel

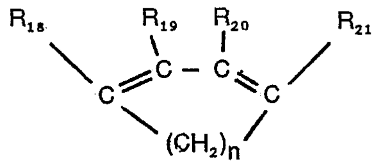

worin $R_{18}$, $R_{19}$, $R_{20}$ und $R_{21}$ unabhängig voneinander Wasserstoff, $C_1$- bis $C_4$-Alkyl, Vinyl, $C_3$- bis $C_7$-Cycloalkyl, oder Phenyl bedeuten und n für eine ganze Zahl von 1 bis 4 steht, einsetzt.

6. Verfahren nach Anspruch 1 bis 5, dadurch gekennzeichnet, daß man als Polyen ein Olefin der Formel

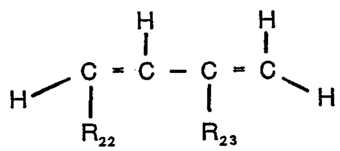

worin $R_{22}$ und $R_{23}$ unabhängig voneinander Wasserstoff, Methyl, Äthyl, Vinyl oder Phenyl bedeuten, einsetzt.

7. Verfahren nach Anspruch 1 bis 6, dadurch gekennzeichnet, daß man als Polyen Butadien, Isopren, Piperylen, Cyclopentadien, 1,3,5-Hexatrien oder 1,3,7-Octatrien einsetzt.

8. Verfahren nach Anspruch 1 bis 7, dadurch gekennzeichnet, daß man als Percarbonsäure Perpropionsäure oder Perisobuttersäure einsetzt.

9. Verfahren nach Anspruch 1 bis 8, dadurch gekennzeichnet, daß man als Lösungsmittel Benzol, Chlorbenzol oder 1,2-Dichlorpropan einsetzt.

10. Verfahren nach Anspruch 1 bis 9, dadurch gekennzeichnet, daß man die Umsetzung bei einem Molverhältnis von Olefin zur Persäure wie 1,5 bis 3:1 durchführt.

## Revendications

1. Procédé de préparation du vinyloxirane et de vinyloxiranes substitués à partir de polyènes et d'acides percarboxyliques, caractérisé en ce que l'on fait réagir un polyène de formule

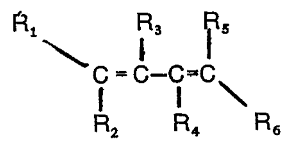

dans laquelle $R_1$, $R_2$, $R_3$, $R_4$, $R_5$ et $R_6$ représentent chacun, indépendamment les uns des autres, l'hydrogène, un groupe alkyle en $C_1$—$C_4$, vinyle, cycloalkyle en $C_3$—$C_7$ ou phényle et $R_1$ et $R_3$, $R_1$ et $R_4$ ou $R_1$ et $R_5$ peuvent former ensemble un noyau carbocyclique contenant jusqu'à 12 atomes de carbone, avec une solution d'un acide percarboxylique contenant 3 à 4 atomes de carbone dans un hydrocarbure aromatique contenant 6 à 12 atomes de carbone ou dans un hydrocarbure chloré contenant 1 à 8 atomes de carbone, qui contient jusqu'à 7% en poids d'eau, jusqu'à 2% en poids de peroxyde d'hydrogène et moins de 50 ppm d'acide minéral, à un rapport molaire polyène/acide percarboxylique de 1,5:1 à 5:1 et à une température allant de −20 à +100°C.

2. Procédé selon la revendication 1, caractérisé en ce que l'on met en oeuvre en tant que polyène une oléfine de formule

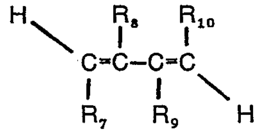

dans laquelle $R_7$, $R_8$, $R_9$ et $R_{10}$ représentent chacun, indépendamment l'un de l'autre, l'hydrogène, un groupe alkyle en $C_1$—$C_4$, vinyle, cycloalkyle en $C_5$—$C_7$ ou phényle, et $R_7$ et $R_8$, $R_7$ et $R_9$ ou $R_7$ et $R_{10}$ peuvent former un noyau carbocyclique contenant jusqu'à 12 atomes de carbone.

3. Procédé selon les revendications 1 et 2, caractérisé en ce que l'on met en oeuvre en tant que polyène une oléfine de formule

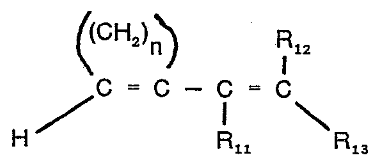

dans laquelle $R_{11}$, $R_{12}$ et $R_{13}$ représentent chacun, indépendamment les uns des autres, l'hydrogène, un groupe alkyle en $C_1$—$C_4$, vinyle, cycloalkyle en $C_3$—$C_5$ ou phényle et n est un nombre entier de 3 à 6.

4. Procéde selon les revendications 1 à 3, caractérisé en ce que l'on met en oeuvre en tant que polyène une oléfine de formule

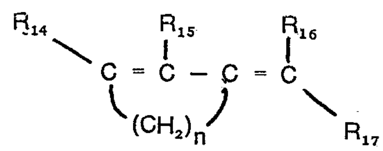

dans laquelle $R_{14}$, $R_{15}$, $R_{16}$ et $R_{17}$ représentent chacun, indépendamment les uns des autres, l'hydrogène, un groupe alkyle en $C_1$—$C_4$, vinyle, cycloalkyle en $C_5$—$C_7$ ou phényle et n est un nombre entier allant de 2 à 5.

5. Procédé selon les revendications 1 à 4, caractérisé en ce que l'on met en oeuvre en tant que polyène un composé de formule

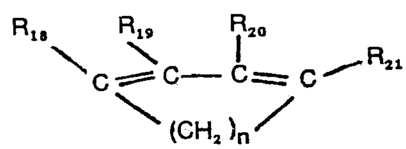

dans laquelle $R_{18}$, $R_{19}$, $R_{20}$ et $R_{21}$ représentent chacun, indépendamment les uns des autres, l'hydrogène, un groupe alkyle en $C_1$—$C_4$, vinyle, cycloalkyle en $C_3$—$C_7$ ou phényle et n est un nombre entier allant de 1 à 4.

6. Procédé selon les revendications 1 à 5, caractérisé en ce que l'on met en oeuvre en tant que polyène une oléfine de formule

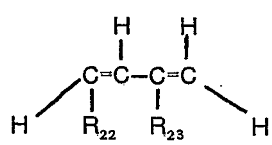

dans laquelle $R_{22}$ et $R_{23}$ représentent chacun, indépendamment l'un de l'autre, l'hydrogène, un groupe méthyle, éthyle, vinyle ou phényle.

7. Procédé selon les revendications 1 à 6, caractérisé en ce que l'on met en oeuvre, en tant que polyène, le butadiène, l'isoprène, le pipérylène, le cyclopentadiène, le 1,3,5-hexatriène ou le 1,3,7-octatriène.

8. Procédé selon les revendications 1 à 7, caractérisé en ce que l'on met en oeuvre en tant qu'acide percarboxylique l'acide perpropionique ou l'acide perisobutyrique.

9. Procédé selon les revendications 1 à 8, caractérisé en ce que l'on utilise comme solvant le benzène, le chlorobenzène ou le 1,2-dichloropropane.

10. Procédé selon les revendications 1 à 9, caractérisé en ce que l'on effectue la réaction à un rapport molaire oléfine/peracide de 1,5 à 3:1.

**Claims**

1. Process for the preparation of vinyloxirane and substituted vinyloxiranes from polyenes and percarboxylic acids, characterised in that a polyene of the formula

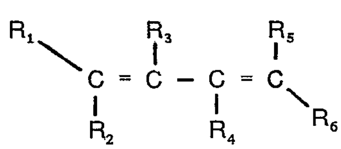

wherein $R_1$, $R_2$, $R_3$, $R_4$, $R_5$ and $R_6$ independently of one another denote hydrogen, $C_1$- to $C_4$-alkyl, vinyl, $C_3$- to $C_7$-cycloalkyl or phenyl,
and wherein $R_1$ and $R_3$, $R_1$ and $R_4$ or $R_1$ and $R_5$ can form a carbocyclic ring with up to 12 carbon atoms, is reacted with a solution of a percarboxylic acid containing 3 to 4 carbon atoms in an aromatic hydrocarbon with 6 to 12 carbon atoms or in a chlorinated hydrocarbon containing 1 to 8 carbon atoms, which solution contains up to 5% by weight of water, up to 2% by weight of hydrogen peroxide and less than 50 ppm of mineral acid, at a molar ratio of polyene to percarboxylic acid of 1.5:1 to 5:1 and at a temperature of −20°C to +100°C.

2. Process according to Claim 1, characterised in that an olefine of the formula

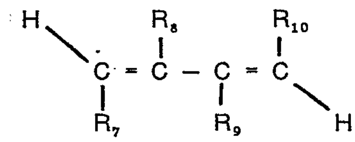

wherein $R_7$, $R_8$, $R_9$ and $R_{10}$ independently of one another denote hydrogen, $C_1$- to $C_4$-alkyl, vinyl, $C_5$- to $C_7$-cycloalkyl or phenyl,
and wherein $R_7$ and $R_8$, $R_7$ and $R_9$, or $R_7$ and $R_{10}$ can form a carbocyclic ring with up to 12 carbon atoms, is employed as the polyene.

3. Process according to Claim 1 and 2, characterised in that an olefine of the formula

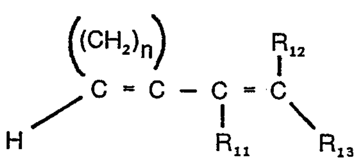

wherein $R_{11}$, $R_{12}$ and $R_{13}$ independently of one another denote hydrogen, $C_1$- to $C_4$-alkyl, vinyl, $C_3$- to $C_5$-cycloalkyl or phenyl and
n represents an integer from 3 to 6,
is employed as the polyene.

4. Process according to Claim 1 to 3, characterised in that an olefine of the formula

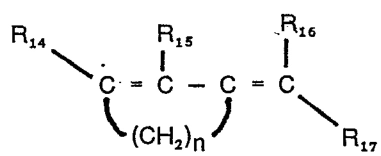

wherein $R_{14}$, $R_{15}$, $R_{16}$ and $R_{17}$ independently of one another denote hydrogen, $C_1$- to $C_4$-alkyl, vinyl, $C_5$- to $C_7$-cycloalkyl or phenyl and
n represents an integer from 2 to 5,
is employed as the polyene.

5. Process according to Claim 1 to 4, characterised in that a compound of the formula

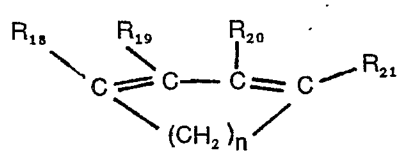

wherein $R_{18}$, $R_{19}$, $R_{20}$ and $R_{21}$ independently of one another denote hydrogen, $C_1$- to $C_4$-alkyl, vinyl, $C_3$- to $C_7$-cycloalkyl or phenyl and
n represents an integer from 1 to 4,
is employed as the polyene.

6. Process according to Claim 1 to 5, characterised in that an olefine of the formula

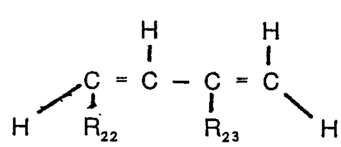

wherein $R_{22}$ and $R_{23}$ independently of one another denote hydrogen, methyl, ethyl, vinyl or phenyl, is employed as the polyene.

7. Process according to Claim 1 to 6, characterised in that butadiene, isoprene, piperylene, cyclopentadiene, 1,3,5-hexatriene or 1,3,7-octatriene are used as the polyene.

8. Process according to Claim 1 to 7, characterised in that perpropionic acid or perbutyric acid are used as the percarboxylic acid.

9. Process according to Claim 1 to 8, characterised in that benzene, chlorobenzene or 1,2-dichloropropane are used as the solvent.

10. Process according to Claim 1 to 9, characterised in that the reaction is carried out at a molar ratio of olefine to peracid of 1.5 to 3:1.